(19) Europäisches Patentamt
European Patent Office
Office européen des brevets

(11) Veröffentlichungsnummer: **0 512 281 A1**

(12) **EUROPÄISCHE PATENTANMELDUNG**

(21) Anmeldenummer: **92106399.6**

(22) Anmeldetag: **14.04.92**

(51) Int. Cl.⁵: **A61M 39/00, A61J 1/00**

(30) Priorität: **02.05.91 DE 4114246**

(43) Veröffentlichungstag der Anmeldung:
**11.11.92 Patentblatt 92/46**

(84) Benannte Vertragsstaaten:
**AT DE FR NL**

(71) Anmelder: **SCHIWA GmbH**
**Kattenvenner Strasse**
**W-4519 Glandorf(DE)**

(72) Erfinder: **Rahimy, Ismael**
**Hirtenstrasse 15**
**W-4500 Osnabrück(DE)**
Erfinder: **Rycyk, Manfred**
**Lortzingstrasse 14**
**W-4516 Bissendorf(DE)**
Erfinder: **Wesseler, Matthias**
**Uphoefener Feld 16**
**W-4517 Hilter 2(DE)**

(54) **Konnektor für einen Behälter für pharmazeutische Lösungen.**

(57) Der Konnektor besteht aus einem ersten, in das Anschlußteil (2) eines Behälters (3) einsteckbaren Kopplungsteil (1) und einem in dieses einsteckbaren zweiten Kopplungsteil (5), welches mit einer Schlauchleitung (8) verbunden ist. Das erste Kopplungsteil ist an der Einstecköffnung (11) mit einem vorperforierten, gummielastischen Stopfen (12) und darüber mit einer Verschlußkappe (15) versehen und nach außen durch einen Abbrechverschluß (17) abgeschlossen.

EP 0 512 281 A1

Die Erfindung betrifft einen Konnektor für einen Behälter für pharmazeutische Lösungen, der mit einem schlauchförmigen Anschlußteil versehen ist, bestehend aus einem in das Anschlußteil einsteckbaren ersten Kopplungsteil mit einem Durchflußkanal und einem in dieses einsteckbaren zweiten Kopplungsteil mit einem Durchflußkanal, welches mit einer Schlauchleitung verbunden ist, und aus einem Absperrorgan.

Aus dem deutschen Gebrauchsmuster G 8812460 ist ein Konnektor für den vorstehend erwähnten Zweck bekannt, der aus einem in das Anschlußteil eines Behälters einsteckbaren Kopplungsteil und einem innerhalb des Anschlußteils sich befindenden Absperrorgan besteht, wobei das Kopplungsteil einen hohlen Einstechdorn aufweist.

Beim Einstechen des Dorns in den gummielastischen Stopfen eines zu konnektierenden Behälters können Materialteilchen ausgestanzt werden, die in den Lösungsstrom eingespült werden und eine damit durchzuführende Behandlung kontaminieren. Nach dem Entferenen des Dorns aus dem Stopfen bei nur teilweisem Verbrauch des Behälterinhalts kann die Einstichstelle undicht sein. Durch das Absperrorgan, beispielsweise einen Abbrechverschluß, im Anschlußteil wird der Durchflußquerschnitt erheblich reduziert, besonders nachteilig bei Operationen oder Behandlungen, bei denen kurzfristig größere Flüssigkeitsmengen benötigt werden.

Es war daher die Aufgabe zu lösen, einen Konnektor zu schaffen, der kontaminationsfrei und mit uneingeschränktem Durchflußquerschnitt zu betreiben ist und bei dem die Konnektion ohne Dichtheitsverlust wieder rückgängig gemacht werden kann.

Gelöst wurde die Aufgabe durch einen Konnektor der eingangs geschilderten Art, bei dem das erste Kopplungsteil an der Einsecköffnung mit einem gummielastischen Stopfen abgeschlossen ist, über dem sich eine dichtende Verschlußkappe befindet, die mit einem deren zentrale Öffnung abschließenden Abbrechverschluß versehen ist, und die zentrale Öffnung Arretierungsvorsprünge für das in diese einsteckbare zweite Kopplungsteil aufweist, an welchem sich mit diesen korrespondierende Vorsprünge befinden.

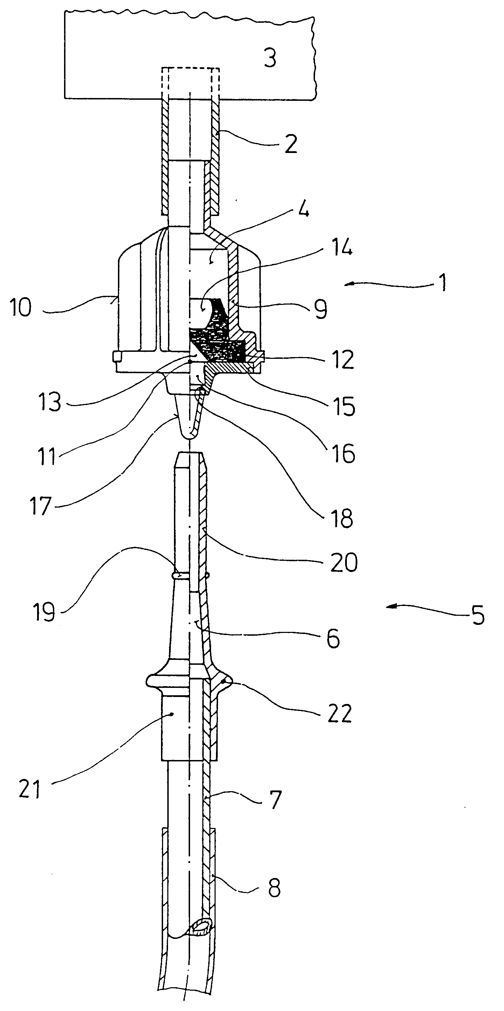

Weitere Einzelheiten und Vorteile des Konnektors nach der Erfindung sind in der nachfolgenden Beschreibung eines in der Zeichnung schematisch dargestellten Ausführungsbeispiels enthalten.

Der Konnektor besteht, wie in der Zeichnung in einem Teilschnitt im nicht-konnektierten Zustand zu sehen, aus einem ersten Kopplungsteil 1, das in das schlauchförmige Anschlußteil 2 eines Behälters 3, beispielsweise eines Beutels, für pharmazeutische Lösungen eingesteckt ist und einen Durchflußkanal 4 aufweist, und aus einem zweiten Kopplungsteil 5 mit einem Durchflußkanal 6, das in das erste Kopplungsteil 1 einsteckbar ist und über dessen abgesetztes freies Ende 7 eine Schlauchleitung 8 geschoben ist.

Das erste Kopplungsteil 1 ist ein zylindrischer Körper 9 mit radial angeordneten Handhabungsflügeln 10, dessen Durchflußkanal 4 an der Einstecköffnung 11 für das zweite Kopplungsteil 5 in zwei Stufen erweitert ist. In der ersten Stufe ist die Öffnung mit einem gummielastischen Stopfen 12 abgeschlossen, der in Form eines Nadeldurchstichs zentral vorperforiert ist. Der Stopfen weist einsteckseitig eine trichterförmige Einführöffnung 13 und rückseitig eine zentrale Ausnehmung 14 für die Materialverdrängung beim eingesteckten zweiten Kopplungsteil 5 auf. Über dem Stopfen befindet sich in der zweiten Öffnungsstufe in dichtendem Sitz eine Verschlußkappe 15, deren zentrale Öffnung 16 mit einem Abbrechverschluß 17 nach außen abgeschlossen ist.

Die zentrale Öffnung 16 der Verschlußkappe 15 ist mit Arretierungsvorsprüngen oder einem umlaufenden Vorsprung 18 für das eingesteckte zweite Kopplungsteil 5 versehen, an welchem damit korrespondierende Vorsprünge 19 gebildet sind. Eine zweckmäßige Ausführungsform besteht darin, daß ein umlaufender Vorsprung durch die Bruchkante des Abbrechverschlusses 17 gebildet wird.

Das zweite Kopplungsteil 5 ist ein rohrförmiges Teil, dessen Einsteckspitze 20 im Durchmesser etwas kleiner bemessen ist als die zentrale Öffnung 16 der Verschlußkappe 15 an den Vorsprüngen 18. Am Übergang von der Spitze zum Handhabungsteil 21, der einen Handhabungskragen 22 aufweist, befindet sich der erwähnte Arretierungsvorsprung 19. Der an den Handhabungsteil sich anschließende Endabschnitt 7 ist, wie bereits oben beschrieben, zum Anschluß einer Schlauchleitung 8 vorgesehen.

Nach dem Abbrechen des Abbrechverschlusses 17 ist das erste Kopplungsteil 1 bereit für die Konnektion mit dem zweiten Kopplungsteil 5.

**Patentansprüche**

1. Konnektor für einen Behälter für pharmazeutische Lösungen, der mit einem schlauchförmigen Anschlußteil versehen ist, bestehend aus einem in das Anschlußteil (2) einsteckbaren ersten Kopplungsteil (1) mit einem Durchflußkanal (4) und einem in dieses einsteckbaren zweiten Kopplungsteil (5) mit einem Durchflußkanal (6), welches mit einer Schlauchleitung (8) verbunden ist, und aus einem Absperrorgan (17), dadurch gekennzeichnet, daß das erste Kopplungsteil (1) an der Einstecköffnung (11) mit einem gummielastischen Stopfen (12) abgeschlossen ist, über dem sich eine dichtende Verschlußkappe (15) befindet, die mit einem deren zentrale Öffnung (16) abschließenden Abbrechverschluß (17) versehen ist, und die zentrale Öffnung Arretierungsvorsprünge (18) für das in diese einsteckbare zweite Kopplungsteil (5) aufweist, an welchem sich mit diesen korrespondierende Vorsprünge (19) befinden.

2. Konnektor nach Anspruch 1, dadurch gekennzeichnet, daß die Vorsprünge (18) umlaufend in Form eines Rings ausgeführt sind.

3. Konnektor nach Anspruch 1 und 2, dadurch gekennzeichnet, daß die Vorsprünge (18) durch die Bruchkante des Abbrechverschlusses (17) gebildet sind.

4. Konnektor nach Anspruch 1 bis 3, dadurch gekennzeichnet, daß der gummielastische Stopfen (12) in Form eines Nadeldurchstichs vorperforiert ist.

5. Konnektor nach Anspruch 1 bis 4, dadurch gekennzeichnet, daß der Stopfen (12) eine zentrale Ausnehmung (14) aufweist.

6. Konnektor nach Anspruch 1 bis 5, dadurch gekennzeichnet, daß der Stopfen (12) eine trichterförmige Einführöffnung (13) für das einsteckbare Kopplungsteil (5) aufweist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | EP-A-0 319 764 (NISSHO CORP) <br> * Abbildung 7B * <br> --- | 1-6 | A61M39/00 <br> A61J1/00 |
| Y | DE-A-3 618 158 (SCHIWA GMBH) <br> * Spalte 2, Zeile 38 - Zeile 67; Abbildung 1 * <br> --- | 1-6 | |
| A | EP-A-0 312 035 (MECT CORP) <br> * Zusammenfassung; Abbildungen * <br> --- | 1-6 | |
| A | US-A-4 354 490 (ROGERS) <br> * Spalte 3, Zeile 53 - Zeile 68; Abbildungen 5-7 * <br> --- | 1-6 | |
| A | FR-A-1 337 891 (LABORATOIRES DU DR DEBAT) <br> * Seite 2, linke Spalte, Zeile 11 - Zeile 28; Abbildung 6 * <br> ----- | 1-6 | |

| RECHERCHIERTE SACHGEBIETE (Int. Cl.5 ) |
|---|
| A61M <br> A61J |

Der vorliegende Recherchenbericht wurde für alle Patentansprüche erstellt

| Recherchenort | Abschlußdatum der Recherche | Prüfer |
|---|---|---|
| DEN HAAG | 27 JULI 1992 | CLARKSON P. |